(19) **Europäisches Patentamt**

**European Patent Office**

**Office européen des brevets**

(11) **EP 0 850 403 B1**

(12) **EUROPÄISCHE PATENTSCHRIFT**

(45) Veröffentlichungstag und Bekanntmachung des Hinweises auf die Patenterteilung:
**14.11.2001 Patentblatt 2001/46**

(21) Anmeldenummer: **96945132.7**

(22) Anmeldetag: **13.09.1996**

(51) Int Cl.7: **G01N 11/08**, G01N 33/44

(86) Internationale Anmeldenummer:
**PCT/DE96/01736**

(87) Internationale Veröffentlichungsnummer:
**WO 97/10492 (20.03.1997 Gazette 1997/13)**

(54) **VERFAHREN ZUM ERMITTELN UND AUSWERTEN VON SCHMELZINDEXWERTEN**

PROCESS FOR DETERMINING AND EVALUATING MELT FLOW INDEX VALUES

PROCEDE PERMETTANT DE DETERMINER ET D'EVALUER DES VALEURS D'INDICE DE FUSION

(84) Benannte Vertragsstaaten:
**BE NL**

(30) Priorität: **13.09.1995 DE 19533859**

(43) Veröffentlichungstag der Anmeldung:
**01.07.1998 Patentblatt 1998/27**

(73) Patentinhaber: **GÖTTFERT WERKSTOFF-PRÜFMASCHINEN GMBH D-74722 Buchen (DE)**

(72) Erfinder: **GÖTTFERT, Axel D-74722 Buchen (DE)**

(74) Vertreter: **Naumann, Ulrich, Dr.-Ing. et al Patentanwälte, Ullrich & Naumann, Luisenstrasse 14 69115 Heidelberg (DE)**

(56) Entgegenhaltungen:
| | |
|---|---|
| **EP-A- 0 406 805** | **EP-A- 0 595 276** |
| **WO-A- 92/17764** | **US-A- 3 048 030** |
| **US-A- 3 252 320** | **US-A- 3 468 158** |
| **US-A- 3 908 442** | **US-A- 4 449 395** |

EP 0 850 403 B1

**Beschreibung**

Die Erfindung betrifft ein Verfahren zum Ermitteln und Auswerten von Schmelzindexwerten (MFR-Werte) von thermoplastischen Materialien mit Hilfe eines kontinuierlich arbeitenden on-line Kapillarrheometers, wobei die Druckdifferenz $\Delta p$ zwischen Eingang und Ausgang einer Meßdüse des Kapillarrheometers vorgegeben wird und $\Delta p$ so geregelt wird, daß

$$MFR_{on\text{-}line} = MFR_{Labor},$$

wobei $MFR_{on\text{-}line}$ der on-line ermittelte Schmelzindexwert ist und $MFR_{Labor}$ der unter standardisierten Laborbedingungen ermittelte Schmelzindexwert ist.

In der US-A-3,252,320 wird eine Vorrichtung zum Bestimmen des Schmelzindexes eines Polymers beschrieben. Dazu wird die Polymerschmelze durch eine Leitung gepumpt, wobei die Temperatur der Leitung und der Polymerschmelze möglichst konstant gehalten wird. Zur Bestimmung des Schmelzindexes wird die Polymerschmelze durch eine Verengung in der Leitung gepumpt. Dabei wird der Druck in der Leitung vor der Verengung gemessen.

In der US-A-3,468,158 wird ein Verfahren zur Bestimmung der Viskosität einer nicht-newtonschen Flüssigkeit beschrieben, bei dem die Flüssigkeit mit definierter Durchflußrate durch eine Leitung mit definiertem Durchmesser gepumpt wird und dabei der Druckabfall über einer definierten Leitungslänge gemessen wird. Diese Messung wird bei unterschiedlichen Meßkonditionen wiederholt. Mit Hilfe von Formeln für laminare Strömungen wird aus den Meßergebnissen dann die Viskosität der Flüssigkeit bestimmt.

In der US-A-4,449,395 wird ein Verfahren zum Testen von thermoplastischen Materialien beschrieben. Im Rahmen dieses Verfahrens wird eine Schmelzprobe entweder bei konstanter Temperatur und konstantem Druck oder bei konstanter Temperatur mit konstanter Durchflußrate durch eine Kontrollzone gepumpt.

Kapillarrheometer sind aufgrund ihres breiten Meßbereichs und ihres einfachen und robusten Aufbaus die am häufigsten in der Polymerindustrie eingesetzten Rheometersysteme.

Eine Möglichkeit der Meßwerterfassung mit einem Kapillarrheometer besteht darin, die Druckdifferenz zwischen Eingang und Ausgang der Meßdüse zu messen, wobei im allgemeinen der Volumenstrom durch die Meßdüse konstant gehalten wird. Ebenso kann aber auch eine konstante Druckdifferenz vorgegeben werden und der sich dadurch ergebende Volumenstrom durch die Meßdüse gemessen werden. Als Meßdüse werden in der Praxis häufig Rundlochdüsen oder Flachschlitzkanäle verwendet. Bei kontinuierlich arbeitenden on-line Kapillarrheometem erfolgt die Dosierung des Volumenstroms mit sogenannten Schmelzepumpen, bspw. Zahnradschmelzepumpen. Der regelbare Arbeitsbereich von Schmelzepumpen liegt zwischen 0,5 und 100 U/min, wobei die Förderkapazität üblicherweise zwischen 0,6 und 1,3 cm$^3$/U liegt. Dadurch kann ein variabler Volumenstrom vorgegeben werden, der es ermöglicht die Viskositätsfunktion der Schmelze in einem spezifizierbaren Bereich abzudecken. Unter der Voraussetzung identischer Düsengeometrien erhält man mit on-line Kapillarrheometern und konventionellen Laborgeräten übereinstimmende Ergebnisse.

Der Schmelzindex (MFR) stellt die populärste Kenngröße zur Beschreibung des Fließverhaltens von Thermoplasten aus industrieller Sicht dar. Auch wenn diese Größe im Rahmen einer klassischen Einpunktmessung ermittelt wird, wird sie von Rohstoffherstellern für die Anwendungstechnik oftmals als einzige rheologische Kennzahl zur Produktspezifizierung angegeben. Insofern kommt dem Schmelzindex MFR auch zentrale Bedeutung bei der produktbegleitenden Qualitätskontrolle und Qualitätssicherung zu.

Der Schmelzindex MFR nach DIN, ISO wird bestimmt als die extrudierte Probenmasse in g, die in zehn Minuten aus einem temperierten Kanal durch eine definierte Düse extrudiert wird, wobei die Probenmasse durch eine definierte Auflage M über einen Kolben belastet wird.

Da sich bei vielen Polymeren die Dichte p der Schmelze nicht signifikant ändert, wurde aus praktischen Gründen auch der Volumenindex (MVR) genormt, bei dem das extrudierte Material in cm$^3$ angegeben wird. Die Bestimmung des Volumenindex MVR stellt die gebräuchlichere Form der Meßwertbestimmung dar.

Die Meßdüse von im Labor eingesetzten Schmelzindexgeräten weist einen Durchmesser von 2,1 mm und eine Länge von 8 mm auf. Die Auflagenmasse wird in Abhängigkeit von dem jeweils zu charakterisierenden Polymer zwischen 2,16 kg und 21,6 kg gewählt.

Im Unterschied zu den im Labor verwendeten Schmelzindexgeräten weisen die Meßdüsen von on-line Kapillarrheometern ein Längen/Durchmesser (L/D)-Verhältnis >10 auf. Diese Konfiguration ist erforderlich, um Druckdifferenzen zwischen dem Eingang und dem Ausgang der Meßdüse in einer Größenordnung zu erzeugen, die mit handelsüblichen Druckaufnehmern erfaßt werden können.

Bei der Messung des MFR bzw. MVR handelt es sich in erster Näherung um einen Kriechversuch, wobei die durch die Auflagemasse auf die Schmelze wirkende Schubspannung $\tau_W$ an der Wand konstant ist.

Bedingt durch die unterschiedlichen Düsengeometrien von im Labor eingesetzten Schmelzindexgeräten und

# EP 0 850 403 B1

on-line Kapillarrheometern und auch bedingt durch die unterschiedlichen jeweils vorliegenden Strömungsverhältnisse ist ein Vergleich zwischen den im Labor ermittelten und on-line ermittelten MFR- bzw. MVR-Werten nur schwer möglich.

Der Erfindung liegt die Aufgabe zugrunde, eine Art Maßstabsübertragung zu ermöglichen, damit die kontinuierlich ermittelten MFR- bzw. MVR-Werte in akzeptablen Grenzen mit den unter standardisierten Laborbedingungen ermittelten MFR- bzw. MVR-Werten übereinstimmen.

Das erfindungsgemäße Verfahren löst die voranstehende Aufgabe durch die Merkmale des Patentanspruchs 1. Danach ist das eingangs genannte Verfahren dadurch gekennzeichnet, daß ein Sollwert für den Druckabfall Δp im Kapillarrheometer bestimmt wird als

$$\Delta p_{on-line} = \frac{2L}{R}\left[ \tau_{MFR} - k(\dot{\gamma}_a^{\,n} - \dot{\gamma}_{MFR}^{\,n}) \right]$$

und solange nicht variiert wird, solange sich das kontinuierlich bestimmte Verhältnis zwischen dem elastischen Spannungsanteil $\tau_{elast.}$ und dem viskosen Spannungsanteil $\tau_{viskos.}$ der Schubspannung $\tau_{MFR}$ nicht wesentlich ändert, wobei

| | |
|---|---|
| L =: | Länge der Meßdüse |
| R =: | Radius der Meßdüse |
| $\tau_{MFR}$ =: | für MFR bei standardisierten Laborbedingungen errechnete Schubspannung |
| k =: | Konsistenz |
| $\dot{\gamma}_a$ =: | für MFR bei standardisierten Laborbedingungen errechnete Schergeschwindigkeit |
| $\dot{\gamma}_{MFR}$ =: | die bei dem MFR im on-line Kapillarrheometer vorliegende Schergeschwindigkeit |
| n =: | Fließexponent. |

Bei der Simulation der Schmelzindexmessung in einem on-line Rheometer muß berücksichtigt werden, daß sich die auf den Kolben einwirkende Gesamtkraft Fgesamt zusammensetzt als

$$F_{gesamt} = F_{viskos} + F_{elast} + F_{Kanal} + F_{KR},$$

wobei

| | |
|---|---|
| $F_{viskos}$ =: | Kraft, die zur Überwindung des Fließwiderstandes in der Meßdüse notwendig ist |
| $F_{elast}$ =: | Kraft zur Überwindung aller Strömungsverluste vor und nach der Meßdüse |
| $F_{Kanal}$ =: | Kraft für den Flüssigkeitstransport im Vorlagekanal |
| $F_{KR}$ =: | Kraft zur Überwindung der Reibung zwischen dem Kolben und dem Vorlagekanal. |

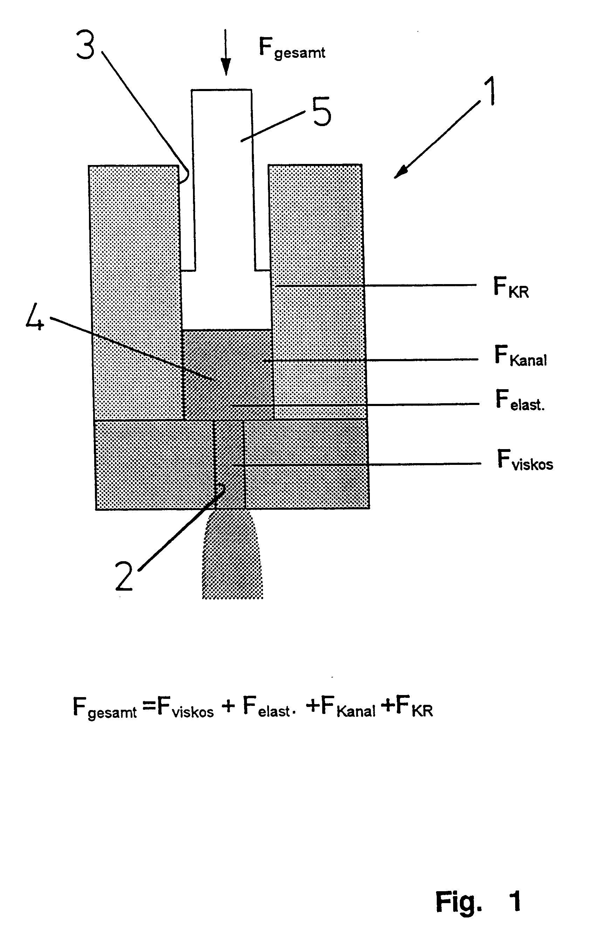

Die Zusammensetzung der Gesamtkraft wird im übrigen auch durch Fig. 1 veranschaulicht, in der schematisch eine standardisierte Meßzelle zur Ermittlung von MFR- bzw. MVR-Werten dargestellt ist.

Die Schubspannung τ, die sich aufgrund der auf den Kolben wirkenden Gesamtkraft $F_{gesamt}$ errechnen läßt, steht demnach nicht vollständig als Druckdifferenz zur Verfügung, um den Fließwiderstand der Meßdüse zu überwinden, da z.B. eine Kolbenkraft $F_{KR}$ aufgebracht werden muß, um die Reibung zwischen Kolben und Vorlagekanal zu überwinden. Eine weitere Kraftkomponente $F_{Kanal}$ ist für den Flüssigkeitstransport im Vorlagekanal erforderlich. Diese Kraftkomponente ist von der Füllhöhe abhängig. Schließlich entfällt noch eine Kraftkomponente $F_{elast}$ auf alle Strömungsverluste vor und nach der Meßdüse, die durch die elastischen Eigenschaften der Schmelze verursacht werden. Aufgrund des kleinen L/D-Verhältnisses der Meßdüse in Laborgeräten kann $F_{elast}$ mitunter betragsmäßig so hoch sein, wie die eigentliche Kraft $F_{viskos}$, die zur Überwindung des Fließwiderstandes in der Meßdüse notwendig ist.

Aus den voranstehenden Überlegungen folgt, daß im spannungsgesteuerten on-line Versuch ausschließlich die Schubspannung $\tau_W$ konstant gehalten wird, die durch die Kraftkomponente $F_{viskos}$ verursacht wird. Diese vereinfachende Annahme kann nur zum Teil die wesentlich komplexeren Strömungsverhältnisse im Laborgeräte wiedergeben. So bleiben die viskoelastischen Effekte, welche sich bspw. durch die Beträge der Einlauf- und Auslaufdruckverluste nachweisen lassen, unberücksichtigt, obwohl diese Effekte aufgrund der Geometrie der Standardmeßdüse ihren Niederschlag in der genormten MFR- bzw. MVR-Messung finden.

Inwiefern eine befriedigende Übereinstimmung von Labor- und on-line Werten erzielt wird, hängt also maßgeblich von den Übertragungsmodellen bzw. den in diesen Übertragungsmodellen festgelegten Randbedingungen ab.

Der MFR-Wert läßt sich aus dem spannungsgesteuerten kontinuierlichen Kapillarrheometerversuch bestimmen als

$$MFR_{on\text{-}line} = MFR_{Labor, Kal.} \cdot \frac{\dot{\gamma}_{on\text{-}line}}{\dot{\gamma}_{Kal.}} \qquad (1)$$

Hierbei wird vorausgesetzt, daß der MFR-Wert des Ausgangsstoffes, $MFR_{Labor,Kal}$ bekannt ist und sich damit die entsprechende Schergeschwindigkeit $\dot{\gamma}_{Kal}$ im MFR-Gerät, also unter standardisierten Laborbedingungen, bestimmen läßt als

$$\dot{\gamma}_{Kal.} = 1{,}833 \cdot \frac{MFR}{\rho} \qquad (2)$$

Unter Vorgabe einer konstanten Schubspannung an der Wand ($\tau_W$ = konstant) führen Produktschwankungen zwangsläufig zu sich ändernden Volumenströmen V, wodurch sich eine aktuelle Schergeschwindigkeit $\dot{\gamma}_{on\text{-}line}$ einstellt, welche direkt proportional zum neuen MFR-Wert ist.

Mit Gleichung (1) läßt sich jedoch nur dann eine akzeptable Übereinstimmung zwischen errechneten und tatsächlich gemessenen Werten erzielen, wenn die Molmassenverteilungen der im Prozeß sich ändernden thermoplastischen Materialien, wie z.B. Polymerchargen, im Verhältnis zum Ausgangsstoff, der ja durch $MFR_{Labor,Kal}$ charakterisiert ist, annähernd gleich sind.

Schmelzen mit ähnlicher Molmassenverteilung lassen sich nach Vinogradov und Malkin auf eine molmasseninvariante Masterviskositätsfunktion zurückführen. Ist diese strenge Randbedingung erfüllt, dann ist das Verhältnis der Schergeschwindigkeiten in Gleichung (1) direkt proportional zu Änderungen der mittleren Molmasse $M_W$. In diesem Falle stimmt der errechnete Wert $MFR_{on\text{-}line}$ mit dem im Labor gemessenen Wert gut überein. Hat sich jedoch die Molmassenverteilung signifikant geändert, so muß ein neuer Kalibrierwert $MFR^*_{Labor,Kal}$ zugrundegelegt werden.

Bislang wurde der im laufenden Prozeß zugrundegelegte Kalibrierwert $MFR_{Labor,Kal}$ anhand von parallel durchgeführten Labormessungen immer wieder auf seine Gültigkeit hin überprüft. Diese Verfahrensweise ist zum einen zeitaufwendig und führt zum anderen auch zu Ungenauigkeiten bei der Schmelzindexbestimmung.

Die Bedingung der rheologischen Ähnlichkeit, die eine erfolgreiche Verschiebung und Masterung der Viskositätsfunktion ermöglicht, ist u.a. dann erfüllt, wenn der Anstieg der in Betracht kommenden Viskositätsfunktionen bei konstanten Spannungen konstant bleibt. Dies läßt sich im on-line Prozeß leicht überprüfen, indem der Fließexponent n bei zwei konstanten Schubspannungen, die nacheinander angesteuert werden, errechnet wird. Eine andere Möglichkeit besteht darin, die Steigung n der kontinuierlich gemessenen Fließkurve bei konstanten Schergeschwindigkeiten zu messen und diese dann mit unterschiedlichen Verzweigungsstrukturen von Polyamiden zu korrelieren.

In einem weiteren Verfahren wird durch Einsatz einer Keilspaltdüse simultan zur Bestimmung des MFR-Wertes nach Gleichung (1) auch der Fließexponent n des mit der Keilspaltdüse erreichbaren Viskositätsspektrums ermittelt. Ändert sich der Fließexponent signifikant, so kann man davon ausgehen, daß sich die Molmassenverteilung geändert hat. In diesem Falle ist eine Neubestimmung der Kalibrierwerte erforderlich. Diese Vorgehensweise hat den Vorteil, daß der Fließexponent n im Bereich konstanter Spannung ermittelt wird, wodurch immer konstantes Fließverhalten vorliegt, so daß der Fließexponent als signifikantes Maß für Änderungen der Molmassenverteilung gewertet werden kann. Vergleicht man hingegen die Fließexponenten unterschiedlicher Schmelzen in Punkten konstanter Schergeschwindigkeit, so können die darin bestimmten Steigungen nicht als repräsentatives Maß für Änderungen der Molmassenverteilung gewertet werden, da bei konstanten Schergeschwindigkeiten im nicht-Newtonschen Bereich immer unterschiedliche Spannungszustände vorliegen.

Mit der vorliegenden Erfindung wird nun ein Verfahren vorgeschlagen, das unabhängig von den voranstehend beschriebenen einschränkenden Voraussetzungen eine akzeptable Übereinstimmung von Labor- und on-line Messungen ermöglicht. Im Rahmen dieses Verfahrens wird auch der Modellansatz für die Übertragung ständig überprüft, wobei die notwendigen Korrekturen der Einstellwerte automatisch durchgeführt werden.

Wie eingangs bereits dargelegt, berücksichtigen die meisten on-line Rheometer ausschließlich die Kraftkomponente $F_{viskos}$, die zur Überwindung des Fließwiderstandes in der Meßdüse notwendig ist und die viskosen Energieanteile repräsentiert, da im Rahmen von on-line Kapillarrheometem Meßdüsen mit einem großen L/D-Verhältnis eingesetzt werden.

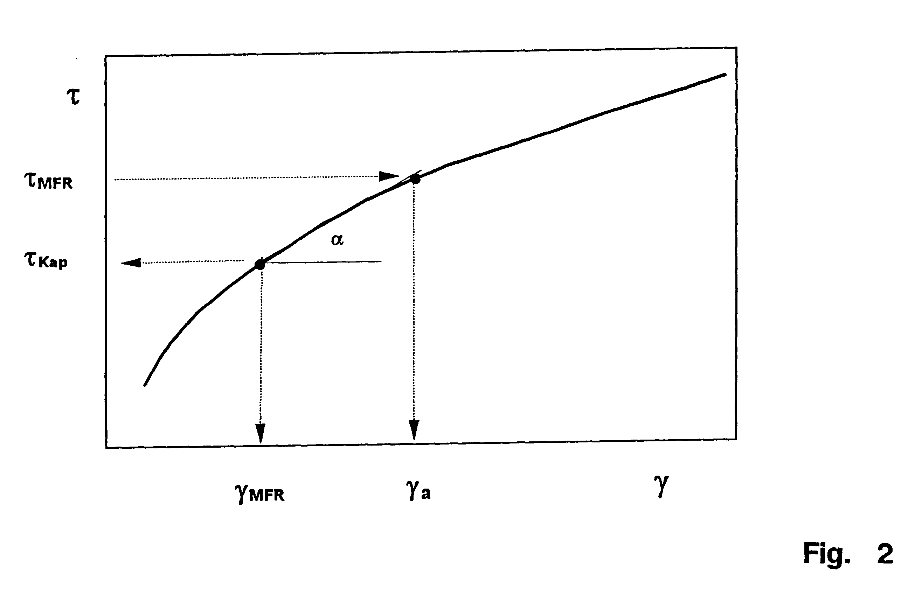

Bei der Ermittlung von Schmelzindexwerten unter standardisierten Laborbedingungen sind jedoch die elastischen Energieverluste nicht zu vernachlässigen, die auf das vergleichsweise kleine L/D-Verhältnis der hier verwendeten Meßdüse zurückzuführen sind. Diese elastischen Energieverluste bedingen, daß die aus dem gewählten Auflagegewicht $F_{gesamt}$ errechnete Schubspannung $\tau_{MFR}$ und die damit verbundene Schergeschwindigkeit $\dot\gamma_a$ immer höher sind als die Schubspannung $\tau_{Kap}$ im Kapillarrheometer, die zum Erzeugen des entsprechenden MFR-Werts notwendig ist. Mit der Schubspannung $\tau_{Kap}$ ist die Schergeschwindigkeit $\dot\gamma_{MFR}$ verbunden. Fig. 2 zeigt den voranstehend beschriebenen Zusammenhang zwischen Schergeschwindigkeit $\gamma$ und Schubspannung $\tau$, der sich in der Fließkurve wiederspiegelt.

Die Steigung der Fließkurve im Bereich $\tau_{MFR}$ - $\tau_{Kap}$ ergibt sich demnach als

$$\tan\alpha = \frac{\tau_{MFR} - \tau_{Kap}}{\dot\gamma_a - \dot\gamma_{MFR}} \qquad (3)$$

nach entsprechender Umformung ergibt sich:

$$\frac{\dot\gamma_{MFR}}{\dot\gamma_a} = 1 - \frac{\tau_{MFR} - \tau_{Kap}}{\tan\alpha \bullet \dot\gamma_a} \qquad (4)$$

Gleichung (4) liefert eine Spannungsanalyse in Form der dimensionslosen Schergeschwindigkeit $\dot\gamma^*$:

$$\dot\gamma^* = \frac{\dot\gamma_{MFR}}{\dot\gamma_a} \qquad (5)$$

Die sich ergebende Schubspannungsdifferenz $\tau_{MFR}$ - $\tau_{Kap}$, die aus der Kräftebilanz des unter Laborbedingungen eingesetzten MFR-Gerätes abgeleitet werden kann, hat die Form:

$$\tau_{MFR} - \tau_{Viskos} = \tau_{elast.} + \tau_{Kanal} + \tau_{Leck} \qquad (6)$$

Da der Energieanteil $\tau_{Leck}$, der für die Leckströmung aufgebracht werden muß, vernachlässigt werden kann, und der Energieverlust $\tau_{Kanal}$ wesentlich kleiner ist als die elastischen Ein- und Auslaufeffekte, kann man die Spannungsdifferenz $\tau_{MFR}$ - $\tau_{Kap}$ in erster Näherung als die elastische Spannungskomponente $\tau_{elast}$ bezeichnen. Damit läßt sich aus Gleichung (4) eine dimensionslose Weißenberg-Zahl Wb ableiten:

$$1 - \frac{\tau_{MFR} - \tau_{Kap}}{\tan\alpha \bullet \dot\gamma_a} = 1 - \frac{\tau_{elast.}}{\tau_{viskos}} = 1 - Wb \qquad (7)$$

Hierbei entspricht $\tau_{Kap}$ in erster Näherung der Spannung $\tau_{viskos}$ nach Gleichung (6). Aus den Gleichungen (5) und (7) folgt somit die dimensionslose Formel:

$$\dot\gamma^* = 1 - Wb \qquad (8)$$

Mit Gleichung (8) werden die auftretenden elastischen und viskosen Spannungsanteile in Beziehung zueinander gebracht. Durch Substitution der Spannungsdifferenz $\tau_{MFR}$ - $\tau_{Kap}$ in den Potenzansatz nach

$$\tau = k \cdot \overset{\cdot}{\gamma}{}^{n}$$

(9)

wobei n dem Fließexponenten und k der Konsistenz entspricht, folgt

$$\tau_{MFR} - \tau_{Kap} = k(\overset{\cdot}{\gamma}{}_{a}^{n} - \overset{\cdot}{\gamma}{}_{MFR}^{n})$$

(10)

In Analogie zu Gleichung (7) ergibt eine weitere Umformung

$$\frac{\overset{\cdot}{\gamma}_{MFR}}{\overset{\cdot}{\gamma}_{a}} = \sqrt[n]{1 - \frac{\tau_{MFR} - \tau_{Kap}}{k \cdot \overset{\cdot}{\gamma}{}_{a}^{n}}} = \sqrt[n]{1 - Wb}$$

(11)

Aus der Schubspannung $\tau_{Kap}$ läßt sich nach weiterer Umformung der Druckabfall $\triangle p$ im on-line Kapillarrheometer bestimmen als

$$\Delta p_{on-line} = \frac{2L}{R}\left[\tau_{MFR} - k(\overset{\cdot}{\gamma}{}_{a}^{n} - \overset{\cdot}{\gamma}{}_{MFR}^{n})\right]$$

(12)

Mit der Gleichung (12) läßt sich unter der Voraussetzung, daß der MFR-Wert bekannt ist und bei quasi konstanten visko-elastischen Spannungsverhältnissen, eine Sollwertvorgabe für den Druckabfall $\triangle p_{on-line}$ im Kapillarrheometer bestimmen. Dieser Druckabfall $\triangle p_{on-line}$ führt zu einem Volumenstrom V durch die Meßdüse, aus dem sich die

6

aktuelle Schergeschwindigkeit $\dot{\gamma}_{on-line}$ und damit wiederum der MFR-Wert nach Gleichung (2) berechnen läßt.

Im Falle einer vorliegenden MFR-Funktion für alle Belastungsstufen i und dem entsprechenden Potenzansatz

$$\tau = k_i \cdot \dot{\gamma}_{MFR}^{i}$$

$$(13)$$

erhält man mit Gleichung (11) die allgemeine Druckgleichung für das on-line Kapillarrheometer

$$\Delta p_{on-line} = \frac{2L}{R} \left\{ \tau_{MFR} \left[ \tau_{MFR} \left( 1 - \frac{\sqrt[i \cdot n]{\frac{\tau_{MFR}}{k_i}}}{\frac{\tau_{MFR}}{k}} \right) \right] \right\}$$

$$(14)$$

Eine Änderung der visko-elastischen Spannungsverhältnisse spiegelt sich in einer wesentlichen Änderung des Weißenberg-Terms wieder. In diesem Falle muß die Spannungsdifferenz $\tau_{MFR}$ - $\tau_{Kap}$ nach Gleichung (11) durch voneinander unabhängige Ausdrücke substituiert werden. Die elastischen Ein- und Auslaufdruckverluste $\tau_{elast}$ nach Gleichung (6) lassen sich durch den Bagley-Term $p_C$ direkt charakterisieren, der bei der Schergeschwindigkeit $\dot{\gamma}_{MFR}$ zu bestimmen ist. Damit gilt

$$\tau_{elast.} = p_c\big|_{\dot{\gamma}_{MFR}} \cdot \left(\frac{R}{2L}\right)_{MFR}$$

$$(16)$$

Aufgrund der Druckverhältnisse in dem im Labor eingesetzten MFR-Gerät ergibt sich eine Schergeschwindigkeit $\dot{\gamma}_{Kanal}$ im Kanal, die etwa 100 mal kleiner ist als die Schergeschwindigkeit in der Meßdüse:

$$\dot{\gamma}_{Kanal} = 0,01063 \cdot \dot{\gamma}_{MFR} \qquad (17)$$

Für viele Polymere gelangt man mit der Schergeschwindigkeit $\dot{\gamma}_{Kanal}$ in den Bereich der Newtonschen Grenzviskosität $\eta_0$. Der Einluß des Füllgrades auf den im Labor-Gerät gemessenen MFR-Wert wird durch den Term $L_Z(t)/L_Z$ berücksichtigt. Damit läßt sich der Energieanteil, der für $\tau_{Kanal}$ aufgebracht werden muß, wie folgt bestimmen:

$$\tau_{Kanal} = 0{,}011063 \cdot \eta_0 \cdot \dot{\gamma}_{MFR} \cdot \frac{L_Z(t)}{L_Z} \tag{18}$$

Die wesentlichen Spannungsverluste, die bei der Schmelzindexmessung auftreten können sind damit bekannt, womit Gleichung (11) neu bestimmt werden kann:

$$\frac{\dot{\gamma}_{MFR}}{\dot{\gamma}_a} = \sqrt[n]{1 - \frac{p_c\Big|_{\dot{\gamma}_{MFR}} \cdot \left(\frac{R}{2L}\right)_{MFR}}{k \cdot \dot{\gamma}_a^{\ n}} + \frac{0{,}01063 \cdot \eta_0 \cdot \dot{\gamma}_{MFR}}{k \cdot \dot{\gamma}_a^{\ n}} \cdot \frac{L_Z(t)}{L_Z}}$$

$$\tag{19}$$

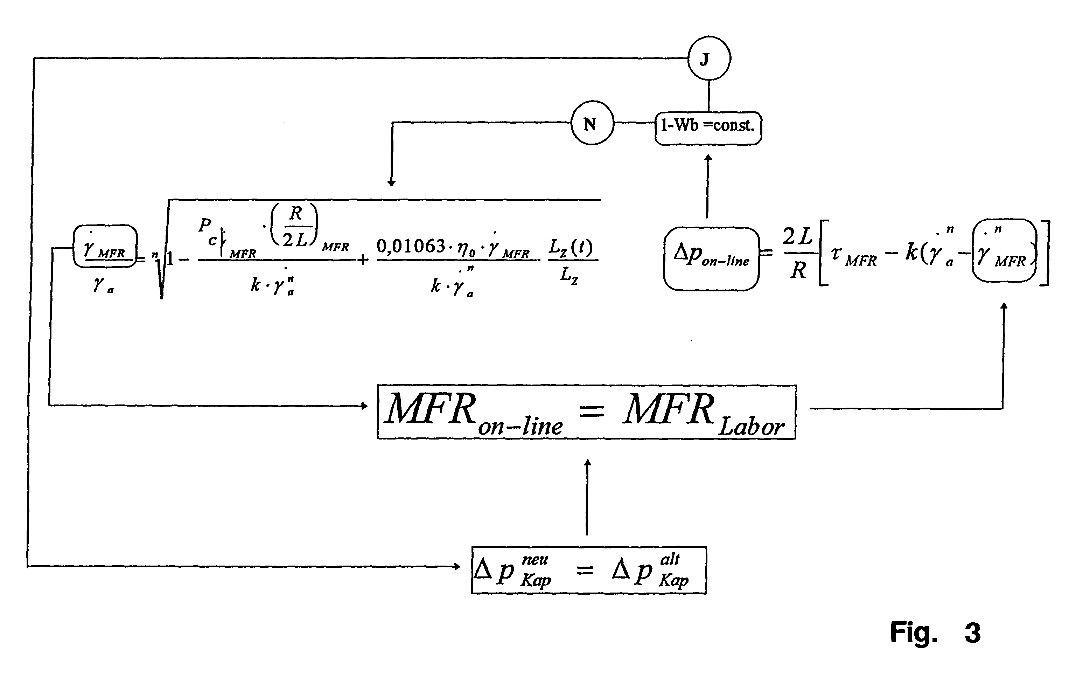

Die einzige unbekannte in Gleichung (19) ist $\dot{\gamma}_{MFR}$, welche sich nun iterativ bestimmen läßt. Damit ist auch der aktuelle Schmelzindex MFR nach Gleichung (2) bestimmt, Fig. 3 zeigt den entsprechenden Algorithmus für ein on-line Kapillarrheometer, mit dem kontinuierlich die Fließeigenschaften einer Schmelze ermittelt und in Form von MFR-Werten ausgewertet werden können, wobei die Meßbedingungen, nämlich $\Delta p$, und damit auch die eigentliche Meßwertauswertung an etwaige Änderungen des visko-elastischen Verhaltens der Schmelze angepaßt werden.

Der Praxiseinsatz des beanspruchten und voranstehend hergeleiteten Algorithmus wird im Rahmen der Figurenbeschreibung in Verbindung mit Fig. 3 näher erläutert.

Der voranstehend beschriebene Algorithmus eines sich selbst korrigierenden on-line Regelkreises verlangt die Ermittlung der Viskositätsfunktion bei mindestens zwei unterschiedlichen Spannungszuständen, damit der Fließexponent n kontinuierlich bestimmt werden kann.

Ändert sich darüber hinaus das visko-elastische Verhalten der zu untersuchenden Schmelze so signifikant, daß der Term (1-WB) nicht mehr konstant bleibt, so ist zusätzlich eine Ermittlung des Bagley-Terms durchzuführen.

Üblicherweise wird die Viskositätsfunktion in on-line Rheometern durch Vorgabe von verschiedenen Schergeschwindigkeiten bzw. unterschiedlichen Schubspannungen ermittelt. Dazu werden nacheinander, zeitversetzt die einzelnen stationären Schergeschwindigkeiten bzw. Schubspannungen vorgegeben. Diese zeitaufwendige Erfassung der Viskositätsfunktion widerspricht den Echtzeitanforderungen eines on-line Rheometers, dessen Aufgabe es ist, unter Echtzeitbedingungen prozeßbegleitende Messungen durchzuführen.

Aus diesem Grunde wurden in der Vergangenheit on-line Rheometer mit mindestens zwei Meßdüsen unterschiedlicher Geometrie eingesetzt. Damit läßt sich bei einem konstanten Volumendurchsatz bzw. konstanter Druckdifferenz ein Bereich der Viskositätsfunktion abdecken. Bekannt ist auch der Einsatz von on-line Rheometern mit zwei in Reihe geschalteten Düsen, deren L/D-Verhältnis im Bereich 3-30 liegt. Auch hier kann bei einem vorgegebenen konstanten Volumendurchsatz ein entsprechender Bereich der Viskositätsfunktion abgedeckt werden.

In einer weiteren Variante werden vier in Reihe geschaltete Düsen mit konstantem L/D-Verhältnis eingesetzt, um bei einem konstant vorgegebenen Volumenstrom einen möglichst großen Bereich der Viskositätsfunktion abzudecken. Entsprechend einem ähnlichen Konzept werden Düsen mit unterschiedlichen Durchmessern parallel geschaltet und mit gleichen Volumenströmen beaufschlagt. Um die elastischen Einlaufeffekte zu eliminieren, können auch zwei in Reihe geschaltete Düsen mit identischen Durchmessern, jedoch unterschiedlichen Längen verwendet werden.

Eine vorteilhafte Variante des erfindungsgemäßen Verfahrens sieht den Einsatz von zwei parallel geschalteten Düsen vor, deren L/D-Verhältnis im Bereich von 10-30 liegt. Des weiteren ist eine Schmelzepumpe vorgesehen, mit der sich die zu fördernde Schmelze in zwei unabhängige und mengenmäßig unterschiedlichen Volumenströme aufteilen läßt. Als Schmelzepumpe käme hier bspw. eine Zahnradplattenpumpe in Frage. Das Verhältnis der beiden Volumenströme sollte im Bereich von 1/10 liegen. Eine mögliche Aufteilung des Gesamtvolumenstroms $V_{ges}$ sieht folgendes qualitatives Verhältnis vor:

$$V_{ges} > V_A >>> V_B \qquad (20)$$

Dadurch ergibt sich ein Fördergrad X für die verbundenen Volumenströme $V_A$ und $V_B$ gemäß:

$$V_{ges} = V_{A(1-X)} + V_{B(X)}$$

$$\text{mit} : 0,1 \leq X \leq 1 \qquad (21)$$

Mit Hilfe der Volumenströme $V_{A(1-X)}$ und $V_{B(X)}$ in Verbindung mit den beiden parallel geschalteten Düsen des on-line Kapillarrheometers läßt sich ein Viskositätsspektrum bei einer konstanten Parametervorgabe, die entweder druck- oder geschwindigkeitsgesteuert erfolgen kann, ermitteln. Daraus sind dann auch der Fließexponent n und die Konsistenz k bestimmbar. Die Erfassung der Viskositätsfunktion erfolgt hier also kontinuierlich im Dauerbetrieb.

Zur Überprüfung der viskoelastischen Grundeinstellung wird im Kurzzeitbetrieb ein Volumenstrom $V_C$ vorgegeben, der bedingt, daß in beiden Düsen vorübergehend eine konstante Schergeschwindigkeit erzielt wird:

$$\dot{\gamma}_A = \dot{\gamma}_B \qquad (22)$$

Da die beiden Düsen unterschiedlich lang sind, läßt sich nun der Bagley-Term $p_C$ berechnen. Zwar stellt Gleichung (22) einen Kompromiß hinsichtlich der klassischen Bagley-Termbestimmung dar, die konstante Düsenradien verlangt. Jedoch ist die einzig mögliche Fehlerquelle, wonach ein Gleiten der Schmelze an der Kapillarwand die Berechnung der Einlaufdruckverluste beeinträchtigen könnte, bei den relativ niedrigen Schergeschwindigkeiten von $\dot{\gamma}_{MFR}$ auszuschließen.

Unter Zugrundelegung der Meßwertanforderungen des erfindungsgemäßen Verfahrens werden folgende Konfigurationen eines Zwei-Düsensystems vorgeschlagen:

1. $V_A = V_B$ sowie: $d_1 = d_2$

Düsen $D_1$ und $D_2$ mit konstantem Durchmesser $d_1$ und $d_2$ und unterschiedlichen Längen $L_1$ und $L_2$ werden mit den parallel geführten Volumenströmen $V_A = V_B$ beschickt. Damit ist $\dot{\gamma}_A = \dot{\gamma}_B$ erfüllt. Kurzzeitig wird ein Volumenstrom $V_C$ vorgegeben, wobei gilt: $V_C \neq (V_A, V_B)$ das führt zu: $\dot{\gamma}_A \neq \dot{\gamma}_B$

2. $V_A = V_B$ sowie: $d_1 \ d_2$

Düsen $D_1$ und $D_2$ mit unterschiedlichen Durchmesser $d_1$ und $d_2$, und unterschiedlichen Längen $L_1$ und $L_2$ werden mit den parallel geführten Volumenströmen $V_A = V_B$ beschickt. Damit ist $\dot{\gamma}_A \neq \dot{\gamma}_B$ erfüllt. Kurzzeitig wird ein Volumenstrom $V_C$ vorgegeben, der dazu führen soll daß: $\dot{\gamma}_A = \dot{\gamma}_B$ erfüllt ist.

3. $V_A \quad V_B$ sowie d: $d_1 = d_2$

Düsen $D_1$ und $D_2$ mit konstantem Durchmesser $d_1$ und $d_2$ und unterschiedlichen Längen $L_1$ und $L_2$ werden mit den parallel geführten Volumenströmen $V_A \neq V_B$ beschickt. Damit ist $\dot{\gamma}_A \neq \dot{\gamma}_B$ erfüllt. Kurzzeitig wird ein Volumenstrom $V_C$ vorgegeben, der dazu führen soll daß: $\dot{\gamma}_A = \dot{\gamma}_B$ erfüllt ist.

4. $V_A \quad V_B$ sowie: $d_1 \quad d_2$

Düsen $D_1$ und $D_2$ mit unterschiedlichen Durchmesser $d_1$ und $d_2$ und unterschiedlichen Längen $L_1$ und $L_2$ werden mit den parallel geführten Volumenströmen $V_A \neq V_B$ beschickt. Damit ist $\dot{\gamma}_A \neq \dot{\gamma}_B$ erfüllt. Kurzzeitig wird ein Volumenstrom $V_C$ vorgegeben, der dazu führen soll daß: $\dot{\gamma}_A = \dot{\gamma}_B$ erfüllt ist.

In Verbindung mit den Figuren werden nachfolgend das erfindungsgemäße Verfahren sowie eine Variante eines on-line Kapillarrheometers beschrieben, das besonders vorteilhaft im Rahmen des erfindungsgemäßen Verfahrens eingesetzt werden kann.

Fig. 1 zeigt den schematischen Aufbau eines im Labor verwendeten Schmelzindex (MFR-Geräts) und dient zur Veranschaulichung der in einem solchen Gerät vorliegenden Kräftebilanz.

Fig. 2 zeigt eine Fließkurve mit den für einen vorgegebenen Schmelzindex-Wert berechneten Schubspannungsverhältnissen und den entsprechenden tatsächlich im on-line Kapillarrheometer vorliegenden Schubspannungsverhältnissen.

Fig. 3 zeigt das Flußdiagramm des im Rahmen des erfindungsgemäßen Verfahrens angewendeten Algorithmus.

Fig. 4 zeigt die Meßzelle eines on-line Kapillarrheometers mit zwei parallel geführten Kapillaren bzw. Meßdüsen unterschiedlicher Länge, die mit unterschiedlichen Volumenströmen beschickt werden.

Fig. 5 zeigt den Drehzahlbereich einer Schmelzepumpe in Abhängigkeit vom MVR bei unterschiedlichen Leistungskennlinien der Pumpe bei einer Düse mit einem L/D-Verhältnis von 60/4.

In Fig. 1 ist die Meßzelle 1 eines Labor-Geräts zumindest schematisch dargestellt, mit dem Schmelzindexwerte unter standardisierten Laborbedingungen ermittelt werden. Wesentlicher Bestandteil der Meßzelle 1 ist eine Meßdüse bzw. Meßkapillare 2, der über einen Vorlagekanal 3 die zu untersuchende Schmelze 4 zugeführt wird. Dazu wird die Schmelze 4 mit einer definierten Auflagekraft belastet. Dies erfolgt über einen im Vorlagekanal 3 auf die Schmelze 4 wirkenden Kolben 5.

Bei der Bestimmung des Schmelzindexes nach DIN, ISO wird die Meßdüse 2 und ggf. auch der Vorlagekanal 3 temperiert. Die Meßdüse 2 hat einen Durchmesser von 2,1 mm und ist 8 mm lang. Die Auflagemasse wird in Abhängigkeit von der zu untersuchenden Schmelze gewählt und liegt in der Größenordnung zwischen 2,16 kg und 21,6 kg.

Die auf dem Kolben 5 lastende Gesamtkraft $F_{gesamt}$ verteilt sich auf mehrere Kraftkomponenten, was in Fig. 1 angedeutet ist.

$$F_{gesamt} = F_{viskos} + F_{elast} + F_{Kanal} + F_{KR}$$

Die Gesamtkraft $F_{gesamt}$ steht also nur teilweise, nämlich mit der Kraftkomponente $F_{viskos}$, zur Überwindung des Fließwiderstandes in der Meßdüse 2 zur Verfügung. Daneben muß noch eine Kraftkomponente $F_{KR}$ aufgebracht werden, um die Reibung zwischen dem Kolben 5 und dem Vorlagekanal 3 zu überwinden. Außerdem ist eine Kraftkomponente $F_{Kanal}$ für den Flüssigkeitstransport im Vorlagekanal 3 notwendig, die wiederum von der Füllhöhe der Schmelze 4 im Vorlagekanal 3 abhängig ist. Schließlich entfällt noch eine Kraftkomponente $F_{elast.}$ auf die Strömungsverluste vor und nach der Meßdüse 2, die durch die elastischen Eigenschaften der Schmelze verursacht werden. Im hier dargestellten Fall eines Laborgeräts kann die Kraftkomponente $F_{elast.}$ mitunter betragsmäßig so hoch sein, wie die Kraftkomponente $F_{viskos}$, die zur Überwindung des Fließwiderstandes in der Meßdüse notwendig ist.

Aus der in Fig. 1 veranschaulichten, in einem Laborgerät vorliegenden Kräftebilanz ergibt sich, daß die aufgrund der auf den Kolben wirkenden Gesamtkraft $F_{gesamt}$ errechnete Schubspannung $\tau_{MFR}$ und die damit verbundene Schergeschwindigkeit $\dot{\gamma}_a$ immer höher ist als die Schubspannung $\tau_{Kap}$ im Kapillarrheometer, die erforderlich ist, um den entsprechenden MFR-Wert zu erzeugen. Dies ist auf die unterschiedlichen L/D-Verhältnisse einerseits an der Meßdüse eines Labor-Geräts und andererseits an der Meßdüse eines Kapillarrheometers zurückzuführen. Aufgrund der unterschiedlichen Geometrien, sind die elastischen Energieverluste bei der Schmelzindexmessung mit einem Kapillarrheometer nämlich in der Regel wesentlich geringer als bei der Bestimmung des Schmelzindexes mit Hilfe eines Laborgeräts. Der Zusammenhang zwischen den Schubspannungsverhältnissen in einem on-line Kapillarrheometer und in einem Laborgerät wird durch die in Fig. 2 dargestellte Fließkurve veranschaulicht.

In Tabelle 1 sind die Ergebnisse von Versuchen wiedergegeben, die in Verbindung mit Gleichung (12) angestellt wurden. Für eine Chargenreihe von Polypropylenen in einem MFR-Bereich von 1-6 g/10 min. wurde der notwen-dige Vorgabedruck $\triangle p_{1-Wb}$ nach Gleichung (12) berechnet. Tabelle 1 zeigt außerdem den experimentell bestimmten Steuerdruck $\triangle p_{on\text{-}line\ Exp}$. Mit Ausnahme der letzten Charge wurden Abweichungen zwischen berechneten und experimentellen Werten gefunden, die unter 5 % lagen.

Einen vergleichbaren Befund ergaben die Berechnungen für die bekannten LDPE- und HDPE Proben in Tabelle 2, wobei die größten Abweichungen zwischen 4 % (1800 S) und 6 % (1840 D) lagen.

Tendenziell sind die Abweichungen zwischen Experiment und Berechnung größer, je kleiner der Weißenberg-Term (1-Wb) ist. Eine Abnahme von (1-Wb) führt in der Regel zu einer Zunahme des elastischen Spannungsanteils. Für die Genauigkeit der Bestimmung der Gleichung (11) ist die exakte Berechnung des Fließexponenten n von größter Bedeutung. Grundsätzlich kann man davon ausgehen, daß für Chargen, deren berechnete Weißenberg-Terme konstant bleiben, sich auch das Spannungsverhältnis $\tau_{MFR\text{-}}\ \tau_{Kap}$ nicht verändert. In diesem Falle kann auch die ursprüngliche Kalibrierung mit $MFR_{Labor,kal.}$ nach Gleichung (1) beibehalten werden.

Tabelle 2 veranschaulicht, daß sich die (1-Wb)-Ausdrücke der PE-Typen in zwei Gruppen einteilen lassen, nämlich in die Gruppe mit $(1\text{-}Wb)_A = 0,61 \pm 0,05$ und in die Gruppe $(1\text{-}Wb)_B = 0,74 \pm 0,05$. Ein Produktwechsel in den jeweiligen Grenzen ist ohne Neukalibrierung möglich.

Ändert sich aber bei einem Chargenwechsel der Term (1-Wb) signifikant, wie bspw. beim Übergang von 3200 K nach 1810 D, so ist davon auszugehen, daß sich auch das Schubspannungsverhältnis $\tau_{elast.}\ /\ \tau_{viskos}$ verändert hat. In diesem Falle wäre also eine Neukalibrierung erforderlich.

In Fig. 3 ist nun der dem erfindungsgemäßen Verfahren zugrundeliegende Algorithmus dargestellt, der eine on-line Ermittlung von Schmelzindexwerten auch unter Berücksichtigung visko-elastischer Spannungsänderungen ermöglicht.

Zum Einstieg in diesen Regelkreis muß der Schmelzindexwert $MFR_{Labor}$ des zu untersuchenden Produktes als Startvorgabe bekannt sein. Mit der Ermittlung der Viskositätsfunktion im Bereich der Schergeschwindigkeit $\dot{\gamma}_{MFR}$ ist dann die Berechnung der Drucksollwertvorgabe als $\Delta p_{on\text{-}line}$ nach Gleichung (12) möglich. Da zu Beginn der Messungen kein Vergleichswert für den Term $(1\text{-}Wb)^{1/n}$ vorliegt, liefert $\triangle p_{on\text{-}line}$ nach Gleichung (1) einen errechneten MFR-Wert, der mit akzeptabler Toleranz mit dem Laborwert übereinstimmt. Solange die kontinuierliche Bestimmung des Weißenberg-Ausdrucks in Grenzen konstant bleibt, kann man davon ausgehen, daß der Sollwert für $\triangle p_{on\text{-}line}$ gute Ergebnisse liefert und also beibehalten werden kann.

Verändert sich jedoch das visko-elastische Verhalten des aktuellen Zustandes der Schmelze in der Weise, daß das Spannungsverhältnis der viskosen und elastischen Spannungsanteile nicht mehr konstant bleibt, so muß der Ausdruck $\tau_{MFR}$ - $\tau_{Kap}$ mit Hilfe von Gleichung (19) direkt berechnet werden. Damit läßt sich dann die aktuelle Schergeschwindigkeit $\dot{\gamma}^{*}_{MFR}$, die sich im Labor-MFR-Gerät einstellen würde, direkt bestimmen, woraus sich wiederum der aktuelle MFR-Wert ergibt. Mit $\dot{\gamma}^{*}_{MFR}$ folgt aus Gleichung (12) ein neuer Sollwertdruck $\triangle p_{on\text{-}line}$, der den veränderten visko-elastischen Zustand der Schmelze berücksichtigt und der gewährleistet, daß die on-line Messung und der Laborwert des MFR in akzeptablen Grenzen übereinstimmen.

Damit liegt ein geschlossener Regelkreis vor, der im Dauerbetrieb einen signifikanten Bereich der Viskositätsfunktion ermittelt und zusätzlich die visko-elastischen Eigenschaften der Schmelze überprüft, um damit die Grundlage für eine prozeßzeitkonforme Bestimmung des MFR zu schaffen.

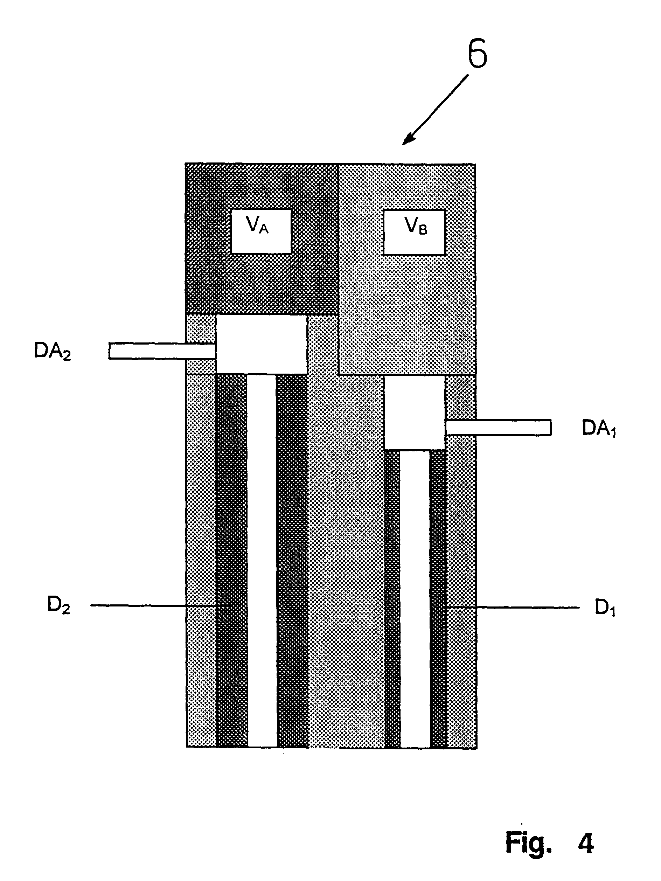

In Fig. 4 ist eine Meßzelle 6 eines on-line Kapillarrheometers dargestellt, das sich besonders gut zur Durchführung des erfindungsgemäßen Verfahrens eignet. Die Meßzelle 6 umfaßt zwei parallel geführte Kapillaren D1 und D2 als Meßdüsen. Die Kapillaren D1 und D2 weisen unterschiedliche Längen auf und werden simultan mit unterschiedlichen Volumenströmen $V_A$ und $V_B$ beaufschlagt. Der sich dabei an der jeweiligen Meßdüse D1 bzw. D2 einstellende Druckabfall wird von den entsprechend angeordneten Druckaufnehmern DA1 und DA2 erfaßt.

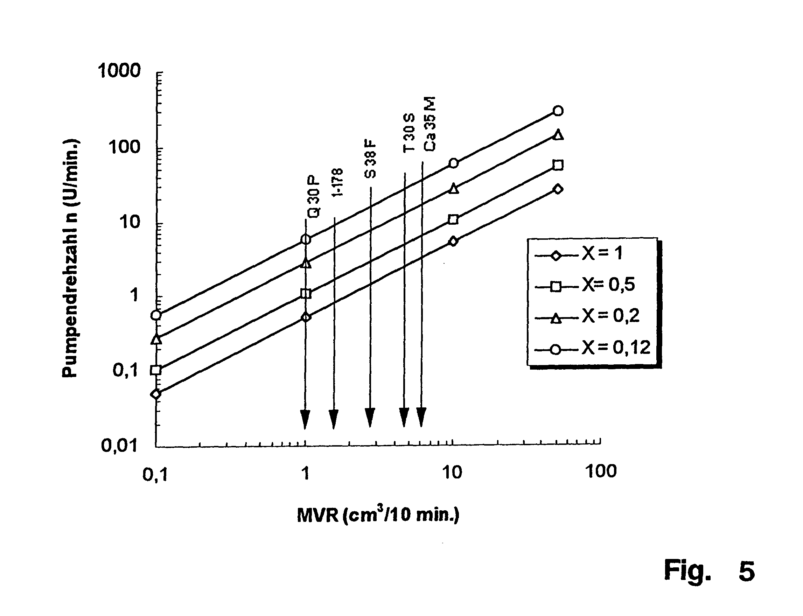

In Fig. 5 ist schließlich noch die Leistungskennlinie einer Spinnpumpe für den Volumenstrom $V_B$ (X) in Abhängigkeit von unterschiedlichen Förderkapazitätsfaktoren X für die bekannten PP-Schmelzen dargestellt. Für die Schmelze mit der Kennlinie X = 1 und einer Düse von 60/4 ergibt sich bspw. ein Drehzahlbereich von etwa 0,4 bis 2 U/min. Drehzahlregelungen unterhalb 1 U/min. sind äußerst kritisch, wobei man bei den beschriebenen Polymeren am Grenzbereich der Rheometerauslegung angelangt wäre.

Sieht man bspw. bei der Auslegung der Pumpe für den Volumenstrom $V_B$ die Fördercharakteristik gemäß Kennlinie X = 0,2 vor, so erhöht sich der Drehzahlbereich insgesamt um ca. das Zehnfache. Damit werden die Bedingungen einer reproduzierbaren und zuverlässigen Regelung der Schmelzpumpe für den hier diskutierten Polymerbereich erst möglich.

| Material | $MVR_{Labor}$ | $\Delta p_{on\text{-}line,\ Exp.}$ | $MVR_{on\text{-}line,\ Exp.}$ | $\Delta p_{(1\text{-}Wb)}^{1/n}$ | $\Delta p_{Exp} - \Delta p_{(1\text{-}Wb)}^{1/n}$ | $1\text{-}Wb$ |
|---|---|---|---|---|---|---|
| PP | | | | | $\Delta\ \%$ | $M_{0=2,16\ kg}$ |
| S 38F | 2,74 | 9,63 | 2,75 | 9,68 | -0,52 | 0,82 |
| T 30S | 4,69 | 9,62 | 4,67 | 9,66 | -0,42 | 0,82 |
| 1-178 | 1,39 | 9,21 | 1,39 | 9,58 | -4,02 | 0,81 |
| Ca 35M | 5,89 | 9,71 | 5,9 | 9,91 | -2,06 | 0,84 |
| Q 30 P | 1,09 | 9,2 | 1,04 | 7,74 | 15,9 | 0,66 |

**Tabelle 4.1** : Berechnete und experimentell bestimmte Drucksollwerte für die kontinuierliche spannungsgesteuerte MFR- Messung unterschiedlicher Polypropylen Sorten.

EP 0 850 403 B1

| Material PE | $MVR_{Labor}$ | $\Delta p_{RTR,}$ Exp. | $\Delta p_{(1-Wb)}^{1/n}$ | $\Delta p_{Exp} - \Delta p_{(1-Wb)}^{1/n}$ $\Delta\%$ | 1-Wb $M_{0=2,16\,kg}$ | Zuordnung |
|---|---|---|---|---|---|---|
| 1800 S | 23,9 | 8,8 | 8,4 | 3,7 | 0,72 | A |
| 2420 K | 5,2 | 7,9 | 7,8 | 1,3 | 0,66 | A |
| 1810 D | 0,31 | 5,7 | 5,5 | 3,5 | 0,59 | A |
| 3020 K | 4,8 | 8,9 | 8,7 | 2,3 | 0,74 | B |
| 1800 H | 1,8 | 7,8 | 7,8 | 0 | 0,66 | A |
| 2420 F | 0,96 | 7,2 | 7,3 | -1,4 | 0,78 | B |
| 1812 E | 0,64 | 6,6 | 6,5 | 1,5 | 0,69 | B |
| 1840 D | 0,3 | 5,0 | 4,7 | 5,6 | 0,5 | |
| 6021 D | 0,3 | 5,2-5,5 | 5,4 | 0 | 0,58 | A |
| 5021 D | 0,27 | 5,2 | 5,2 | 0 | 0,56 | A |

**Tabelle 4.2** : Berechnete und experimentell bestimmte Drucksollwerte für die kontinuierliche spannungsgesteuerte MFR- Messung von Polyethylen.

EP 0 850 403 B1

**Patentansprüche**

1. Verfahren zum Ermitteln und Auswerten von Schmelzindexwerten (MFR-Werte) von thermoplastischen Materialien mit Hilfe eines kontinuierlich arbeitenden on-line Kapillarrheometers, wobei die Druckdifferenz $\Delta p$ zwischen Eingang und Ausgang einer Meßdüse des Kapillarrheometers vorgegeben wird und $\Delta p$ so geregelt wird, daß

$$\text{MFR}_{\text{on-line}} = \text{MFR}_{\text{Labor}},$$

wobei $\text{MFR}_{\text{on-line}}$ der on-line ermittelte Schmelzindexwert ist und $\text{MFR}_{\text{Labor}}$ der unter standardisierten Laborbedingungen ermittelte Schmelzindexwert ist,
**dadurch gekennzeichnet, daß** ein Sollwert für den Druckabfall $\Delta p$ im Kapillarrheometer bestimmt wird als

$$\Delta p_{on-line} = \frac{2L}{R}\left[\tau_{MFR} - k(\dot{\gamma}_a^n - \dot{\gamma}_{MFR}^n)\right]$$

und solange nicht variiert wird, solange sich das kontinuierlich bestimmte Verhältnis zwischen dem elastischen Spannungsanteil $\tau_{\text{elast.}}$ und dem viskosen Spannungsanteil $\tau_{\text{viskos.}}$ der Schubspannung $\tau_{\text{MFR}}$ nicht wesentlich ändert, wobei

L =: Länge der Meßdüse
R =: Radius der Meßdüse
$\tau_{\text{MFR}}$ =: für MFR bei standardisierten Laborbedingungen errechnete Schubspannung
k =: Konsistenz
$\dot{\gamma}_a$ =: für MFR bei standardisierten Laborbedingungen errechnete Schergeschwindigkeit
$\dot{\gamma}_{\text{MFR}}$ =: die bei dem MFR im on-line Kapillarrheometer vorliegende Schergeschwindigkeit
n =: Fließexponent.

2. Verfahren nach Anspruch 1, **dadurch gekennzeichnet, daß** das Verhältnis zwischen dem elastischen und dem viskosen Spannungsanteil der Schubspannung ermittelt wird als

$$\frac{\dot{\gamma}_{MFR}}{\dot{\gamma}_a} = 1 - \frac{\tau_{MFR} - \tau_{Kap}}{\tan \alpha \cdot \dot{\gamma}_a} = 1 - \frac{\tau_{elast.}}{\tau_{viskos}} = 1 - Wb$$

wobei

$\tau_{\text{Kap}}$ =: Schubspannung im Kapillarrheometer zur Erzeugung des MFR
$\tan \alpha$ =: Steigung der Fließkurve im Bereich $\tau_{\text{MFR}} - \tau_{\text{Kap}}$
Wb =: dimensionslose Weißenberg-Zahl.

3. Verfahren nach Anspruch 2, **dadurch gekennzeichnet, daß** das Verhältnis zwischen dem elastischen und dem viskosen Spannungsanteil der Schubspannung neu ermittelt wird als

$$\frac{\dot{\gamma}_{MFR}}{\dot{\gamma}_a} = \sqrt[n]{1 - \frac{p_c\big|_{\dot{\gamma}_{MFR}} \cdot \left(\frac{R}{2L}\right)_{MFR}}{k \cdot \dot{\gamma}_a^{\;n}} + \frac{0,01063 \cdot \eta_0 \cdot \dot{\gamma}_{MFR}}{k \cdot \dot{\gamma}_a^{\;n}} \cdot \frac{L_z(t)}{L_z}}$$

(19)

wenn sich das als

$$\frac{\dot{\gamma}_{MFR}}{\dot{\gamma}_a} = 1 - \frac{\tau_{MFR} - \tau_{Kap}}{\tan\alpha \bullet \dot{\gamma}_a} = 1 - \frac{\tau_{elast.}}{\tau_{viskos}} = 1 - Wb$$

kontinuierlich bestimmte Verhältnis zwischen dem elastischen Spannungsanteil $\tau_{elast.}$ und dem viskosen Spannungsanteil $\tau_{viskos.}$ der Schubspannung $\tau_{MFR}$ wesentlich geändert hat, wobei

$p_C/\dot{\gamma}_{MFR}$ =:     Bagley-Term bestimmt bei der Schergeschwindigkeit $\dot{\gamma}_{MFR}$

$\eta_0$ =:     Newtonsche Grenzviskosität

$L_z(t)/L_z$ =:     Einfluß des Füllgrades auf den unter standardisierten Laborbedingungen gemessenen MFR,

und daß das auf diese Weise neu ermittelte Verhältnis zwischen dem elastischen und dem viskosen Spannungsanteil der Schubspannung als neuer Referenzwert für die nachfolgenden Messungen dient.

4.  Verfahren nach Anspruch 3, **dadurch gekennzeichnet, daß** $\Delta$p neu bestimmt wird als

$$\Delta p_{on-line} = \frac{2L}{R}\left[\tau_{MFR} - k(\dot{\gamma}_a^{\;n} - \dot{\gamma}_{MFR}^{\;n})\right]$$

sobald das kontinuierlich bestimmte Verhältnis zwischen dem elastischen Spannungsanteil $\tau_{elast.}$ und dem viskosen Spannungsanteil $\tau_{viskos.}$ der Schubspannung $\tau_{MFR}$ wesentlich von dem ermittelten Referenzwert abweicht.

5.  Verfahren nach einem der Ansprüche 1 bis 4, **dadurch gekennzeichnet, daß** zu Beginn der on-line Messungen der Schmelzindexwert $MFR_{Labor}$ des zu überwachenden thermoplastischen Materials und die Viskositätsfunktion im Bereich $\dot{\gamma}_{MFR}$ bestimmt werden und daß damit eine erste Drucksollwertvorgabe ermittelt wird als

$$\Delta p_{on-line} = \frac{2L}{R}\left[\tau_{MFR} - k(\dot{\gamma}_a^{\;n} - \dot{\gamma}_{MFR}^{\;n})\right]$$

6.  Verfahren nach einem der Ansprüche 1 bis 5, **dadurch gekennzeichnet, daß** ein on-line Kapillarrheometer mit zwei parallel geschalteten Meßdüsen (D1, D2) und einer Pumpe zum Fördern des thermoplastischen Materials verwendet wird, wobei die Meßdüsen (D1, D2) jeweils ein Längen/Durchmesser (L/D) -Verhältnis im Bereich von 10-30 aufweisen und die Pumpe das thermoplastische Material in zwei unabhängige und mengenmäßig unterschiedliche Volumenströme (VA, VB) aufteilt, so daß das Verhältnis der Volumenströme (VA/VB) im Bereich von 1/10 liegt.

7. Verfahren nach Anspruch 6, **dadurch gekennzeichnet, daß** die beiden Düsen (D1, D2) denselben Durchmesser (d1 = d2) aber unterschiedliche Längen (L1, L2) aufweisen und daß die beiden Düsen (D1, D2) mit gleichen Volumenströmen (VA = VB) beschickt werden.

8. Verfahren nach Anspruch 6, **dadurch gekennzeichnet, daß** die beiden Düsen (D1, D2) unterschiedliche Durchmesser (d1, d2) und unterschiedliche Längen (L1, L2) aufweisen und daß die beiden Düsen (D1, D2) mit gleichen Volumenströmen (VA = VB) beschickt werden.

9. Verfahren nach Anspruch 6, **dadurch gekennzeichnet, daß** die beiden Düsen (D1, D2) denselben Durchmesser (d1 = d2) aber unterschiedliche Längen (L1, L2) aufweisen und daß die beiden Düsen (D1, D2) mit unterschiedlichen Volumenströmen (VA, VB) beschickt werden.

10. Verfahren nach Anspruch 6, **dadurch gekennzeichnet, daß** die beiden Düsen (D1, D2) unterschiedliche Durchmesser (d1, d2) und unterschiedliche Längen (L1, L2) aufweisen und daß die beiden Düsen (D1, D2) mit unterschiedlichen Volumenströmen (VA = VB) beschickt werden.

## Claims

1. Process for ascertaining and evaluating melt-flow index values (MFR values) of thermoplastic materials using a continuously operating on-line capillary rheometer, the pressure difference $\Delta p$ between the inlet and outlet of a measuring nozzle of the capillary rheometer being predetermined and $\Delta p$ being so regulated that

$$MFR_{on\text{-}line} = MFR_{laboratory},$$

in which $MFR_{on\text{-}line}$ is the melt-flow index value ascertained on-line and $MFR_{laboratory}$ is the melt-flow index value ascertained under standardised laboratory conditions,
**characterised in that** a set value for the pressure drop $\Delta p$ in the capillary rheometer is determined as

$$\Delta p_{on\text{-}line} = \frac{2L}{R}\left[\tau_{MFR} - k(\dot{\gamma}_a^n - \dot{\gamma}_{MFR}^n)\right]$$

and is not varied as long as the continuously determined ratio between the elastic stress portion $\tau_{elast.}$ and the viscous stress portion $\tau_{viscos.}$ of the shearing stress $\tau_{MFR}$ does not change substantially, in which

| | |
|---|---|
| $L =$: | length of the measuring nozzle |
| $R =$: | radius of the measuring nozzle |
| $\tau_{MFR} =$: | shearing stress calculated for MFR under standardised laboratory conditions |
| $k =$: | consistency |
| $\dot{\gamma}_a =$: | shear rate calculated for MFR under standardised laboratory conditions |
| $\dot{\gamma}_{MFR} =$: | the shearing rate present at the MFR in the on-line capillary rheometer |
| $n =$: | flow exponent. |

2. Process according to claim 1, **characterised in that** the ratio between the elastic and the viscous stress portion of the shearing stress is ascertained as

$$\frac{\dot{\gamma}_{MFR}}{\dot{\gamma}_a} = 1 - \frac{\tau_{MFR} - \tau_{Kap}}{\tan \alpha \cdot \gamma_a} = 1 - \frac{\tau_{elast.}}{\tau_{viscos}} = 1 - Wb$$

in which

$\tau_{Cap}$ =: shearing stress in the capillary rheometer for the generation of the MFR
tan $\alpha$ =: rise of the flow curve in the region $\tau_{MFR}$-$\tau_{Cap}$
Wb =: dimensionless Weißenberg number.

**3.** Process according to claim 2, **characterised in that** the ratio between the elastic and the viscous stress portion of the shearing stress is freshly ascertained as

$$\frac{\dot{\gamma}_{MFR}}{\dot{\gamma}_a} = \sqrt[n]{1 - \frac{p_c\Big|_{\dot{\gamma}_{MFR}} \cdot \left(\frac{R}{2L}\right)_{MFR}}{k \cdot \dot{\gamma}_a^{\,n}} + \frac{0{,}01063 \cdot \eta_0 \cdot \dot{\gamma}_{MFR}}{k \cdot \dot{\gamma}_a^{\,n}} \cdot \frac{L_Z(t)}{L_Z}}$$

(19)

when the ratio, determined continuously as

$$\frac{\dot{\gamma}_{MFR}}{\dot{\gamma}_a} = 1 - \frac{\tau_{MFR} - \tau_{Kap}}{\tan \alpha \bullet \dot{\gamma}_a} = 1 - \frac{\tau_{elast.}}{\tau_{viscos}} = 1 - Wb$$

between the elastic stress portion $\tau_{elast.}$ and the viscous stress portion $\tau_{viscos.}$ of the shearing stress $\tau_{MFR}$ has changed substantially, in which

$p_C/\dot{\gamma}_{MFR}$ =: Bagley term determined at the shearing rate $\dot{\gamma}_{MFR}$
$\eta_0$ =: Newton limit viscosity
$L_z(t)/L_z$ =: influence of the degree of filling on the MFR measured under standardised laboratory conditions,

and **in that** the ratio, freshly ascertained **in that** manner, between the elastic and the viscous stress portion of the shearing stress is used as a new reference value for subsequent measurements.

**4.** Process according to claim 3, **characterised in that** $\Delta p$ is freshly determined as

$$\Delta p_{on-line} = \frac{2L}{R}\left[\tau_{MFR} - k(\dot{\gamma}_a^{\,n} - \dot{\gamma}_{MFR}^{\,n})\right]$$

as soon as the continuously determined ratio between the elastic stress portion $\tau_{elast.}$ and the viscous stress portion $\tau_{viscos.}$ of the shearing stress $\tau_{MFR}$ deviates substantially from the ascertained reference value.

**5.** Process according to any one of claims 1 to 4, **characterised in that**, at the beginning of the on-line measurements, the melt-flow index value $MFR_{laboratory}$ of the thermoplastic material to be monitored and the viscosity function in the range $\dot{\gamma}_{MFR}$ are determined and **in that** a first pressure set point input is ascertained therewith as

$$\Delta p_{on-line} = \frac{2L}{R}\left[\tau_{MFR} - k(\dot{\gamma}_a^n - \dot{\gamma}_{MFR}^n)\right]$$

**6.** Process according to any one of claims 1 to 5, **characterised in that** there is used an on-line capillary rheometer having two measuring nozzles (D1, D2) connected in parallel and having a pump for conveying the thermoplastic material, the measuring nozzles (D1, D2) each having a length/diameter (L/D) ratio in the range of from 10 to 30, and the pump dividing the thermoplastic material into two independent and quantitatively different volume flows (VA, VB) so that the ratio of the volume flows (VA/VB) lies in the range of 1/10.

**7.** Process according to claim 6, **characterised in that** the two nozzles (D1, D2) have the same diameter (d1 = d2) but different lengths (L1, L2) and **in that** the two nozzles (D1, D2) are charged with the same volume flows (VA = VB).

**8.** Process according to claim 6, **characterised in that** the two nozzles (D1, D2) have different diameters (d1, d2) and different lengths (L1, L2) and **in that** the two nozzles (D1, D2) are charged with the same volume flows (VA = VB).

**9.** Process according to claim 6, **characterised in that** the two nozzles (D1, D2) have the same diameter (d1 = d2) but different lengths (L1, L2) and **in that** the two nozzles (D1, D2) are charged with different volume flows (VA, VB).

**10.** Process according to claim 6, **characterised in that** the two nozzles (D1, D2) have different diameters (d1, d2) and different lengths (L1, L2) and **in that** the two nozzles (D1, D2) are charged with different volume flows (VA = VB).

**Revendications**

**1.** Procédé pour détecter et évaluer des valeurs d'indices de fusion (valeurs MFR) de matériaux thermoplastiques à l'aide d'un rhéomètre capillaire travaillant en ligne en continu, la différence de pression $\Delta p$ entre entrée et sortie d'une buse de mesure du rhéomètre capillaire étant prédéfinie et $\Delta p$ étant régulée de telle manière que

$$MFR_{en\ ligne} = MFR_{laboratoire},$$

$MFR_{en\ ligne}$ étant la valeur de l'indice de fusion déterminée en ligne et $MFR_{laboratoire}$ la valeur de l'indice de fusion déterminée par des conditions de laboratoire standardisées, **caractérisé en ce qu'**on détermine une valeur de consigne pour la chute de pression $\Delta p$ dans le rhéomètre capillaire sous la forme

$$\Delta p_{en\ ligne} = \frac{2L}{R}\left[\tau_{MFR} - k(\dot{\gamma}_a^n - \dot{\gamma}_{MFR}^n)\right]$$

et qu'elle n'est pas modifiée tant que le rapport déterminé en continu entre la composante de contrainte élastique $\tau_{élast.}$ et la composante de contrainte visqueuse $\tau_{visqu.}$ de la contrainte de cisaillement $\tau_{MFR}$ ne se modifie sensiblement pas, où

L = longueur de la buse de mesure
R = rayon de la buse de mesure
$\tau_{MFR}$ = contrainte de cisaillement mesurée pour MFR dans des conditions de laboratoire standardisées
k = consistance

$\dot{\gamma}_a$ = vitesse de cisaillement calculée pour MFR dans des conditions de laboratoire standardisées

$\dot{\gamma}_{MFR}$ = la vitesse de cisaillement présente pour MFR dans le rhéomètre capillaire en ligne

n = exposant d'écoulement.

2. Procédé selon la revendication 1,
**caractérisé en ce que** le rapport entre les composantes de contrainte élastique et visqueuse de la contrainte de cisaillement est déterminé par

$$\frac{\dot{\gamma}_{MFR}}{\dot{\gamma}_a} = 1 - \frac{\tau_{MFR} - \tau_{Kap}}{\tan \alpha \bullet \dot{\gamma}_a} = 1 - \frac{\tau_{elast.}}{\tau_{visqu.}} = 1 - Wb$$

où

$\tau_{Kap}$ = contrainte de cisaillement dans le rhéomètre capillaire pour produire la MFR

$\tan \alpha$ = pente de la courbe d'écoulement dans la zone $\tau_{MFR} - \tau_{Kap}$

Wb = nombre de Weissenberg sans dimension.

3. Procédé selon la revendication 2,
**caractérisé en ce que** le rapport entre les composantes de contrainte élastique et visqueuse de la contrainte de cisaillement est déterminé à nouveau par

$$\frac{\dot{\gamma}_{MFR}}{\dot{\gamma}_a} = \sqrt[n]{1 - \frac{p_c\big|_{\dot{\gamma}_{MFR}} \cdot \left(\frac{R}{2L}\right)_{MFR}}{k \cdot \dot{\gamma}_a^{\,n}} + \frac{0{,}01063 \cdot \eta_0 \cdot \dot{\gamma}_{MFR}}{k \cdot \dot{\gamma}_a^{\,n}} \cdot \frac{L_z(t)}{L_z}}$$

$$(19)$$

lorsque le rapport déterminé en continu par

$$\frac{\dot{\gamma}_{MFR}}{\dot{\gamma}_a} = 1 - \frac{\tau_{MFR} - \tau_{Kap}}{\tan \alpha \bullet \dot{\gamma}_a} = 1 - \frac{\tau_{élast.}}{\tau_{visqu.}} = 1 - Wb$$

entre la composante de contrainte élastique $\tau_{elast.}$ et la composante de la contrainte de visqueuse $\tau_{visq.}$ de la contrainte de cisaillement $\tau_{MFR}$ s'est modifié substantiellement, où

$Pc/\dot{\gamma}_{MFR}$ = terme de Bagley déterminé par la vitesse de cisaillement $\dot{\gamma}_{MFR}$

$\eta O$ = viscosité limite newtonienne

$L_z(t)/L_z$ = influence du taux de remplissage sur le MFR mesuré dans des conditions de laboratoire standardisées

et que ce rapport déterminé à nouveau de cette manière entre les composantes de contrainte élastique et visqueuse de la contrainte de cisaillement sert de nouvelle valeur de référence pour les mesures ultérieures.

4. Procédé selon la revendication 3,
**caractérisé en ce que** $\Delta p$ est déterminée à nouveau par

$$\Delta p_{\text{en ligne}} = \frac{2L}{R}\left[\tau_{MFR} - k(\dot{\gamma}_a^n - \dot{\gamma}_{MFR}^n)\right]$$

aussitôt que le rapport mesuré en continu entre la composante de contrainte élastique $\tau_{\text{élast}}$ et la composante de contrainte visqueuse $\tau_{\text{visq}}$. de la containte de cisaillement s'écarte substantiellement de la valeur de référence détectée.

5. Procédé selon l'une des revendications 1 à 4,
   **caractérisé en ce que**, au début des mesures en ligne, on détermine la valeur d'indice de fusion $MFR_{\text{laboratoire}}$ du matériau thermoplastique à contrôler et la fonction de viscosité dans la zone $\dot{\gamma}_{MFR}$ et qu'on détecte ainsi une première prévision de valeur de consigne de pression par

$$\Delta p_{\text{en ligne}} = \frac{2L}{R}\left[\tau_{MFR} - k(\dot{\gamma}_a^n - \dot{\gamma}_{MFR}^n)\right]$$

6. Procédé selon l'une des revendications 1 à 5,
   **caractérisé en ce qu'**on utilise un rhéomètre capillaire en ligne avec deux buses de mesure (D1, D2) montées en parallèle et une pompe pour transporter le matériau thermoplastique, les buses de mesure (D1, D2) présentant respectivement un rapport longueur/diamètre (L/D) dans la plage de 10 à 30 et la pompe divise le matériau thermoplastique en deux courants volumiques (VA, VB) indépendants et différents quantitativement, de telle sorte que le rapport des courants volumiques (VA/VB) est dans la plage de 1 à 10.

7. Procédé selon la revendication 6,
   **caractérisé en ce que** les deux buses (D1,D2) présentent le même diamètre ($d_1=d_2$) mais des longueurs (L1, L2) différentes et que les deux buses (D1,D2) sont alimentées par des courants volumiques égaux (VA = VB).

8. Procédé selon la revendication 6,
   **caractérisé en ce que** les deux buses D1,D2) présentent des diamètres ($d_1,d_2$) différents et des longueurs ($L_1, L_2$) différentes et que les deux buses (D1,D2) sont alimentées par des courants volumiques égaux (VA=VB).

9. Procédé selon la revendication 6,
   **caractérisé en ce que** les deux buses (D1,D2) présentent le même diamètre ($d_1=d_2$) mais des longueurs (L1,L2) différentes et que les deux buses (D1,D2) sont alimentées par des courants volumiques (VA,VB) différents.

10. Procédé selon la revendication 6,
    **caractérisé en ce que** les deux buses (D1,D2) présentent des diamètres ($d_1,d_2$) différents et des longueurs (L1,L2) différentes et que les deux buses (D1,D2) sont alimentées par des courants volumiques (VA,VB) différents.

$$F_{gesamt} = F_{viskos} + F_{elast.} + F_{Kanal} + F_{KR}$$

Fig. 1

Fig. 3

Fig. 4

Fig. 5